# EUROPEAN PATENT SPECIFICATION

(11) **EP 0 350 455 B1**
(45) Date of publication and mention of the grant of the patent: **12.04.1995**
(21) Application number: 89830188.2
(22) Date of filing: 04.05.1989
(51) Int. Cl.: F17B 1/26, F17B 1/12

(54) **A container for gas in which the pressure is maintained constant when the amount, therein contained, changes, specifically suited for accumulating biogas generated from waste waters and biological sludges**
Gasbehälter mit gleichbleibendem Druck bei sich ändernder Füllmenge, insbesondere für aus Abwässern oder aus festen Massen hergestelltes Biogas
Récipient pour gaz, dans lequel la pression est maintenue constante quand la quantité qu'il contient change, spécialement adapté pour accumuler le gaz biologique généré à partir d'eaux usées et de tas de solides

(30) Priority: 06.06.1988 IT 4793088
(43) Date of publication of application: 10.01.1990
(73) Proprietor: Ente per le nuove tecnologie, l'energia e l'ambiente (ENEA), I-00198 Roma (IT); AGRISILOS S.r.l., I-26039 Vescovato Di Cremona (IT)
(72) Inventor: Tilche, Andrea, I-40033 Casalecchio di Reno (Bologna) (IT); Spedini, Luigi, I-26100 Cremona (IT)
(74) Representative: Sarpi, Maurizio

(56) References cited:
- FR-A- 1 547 683
- FR-A- 2 353 790
- GB-A- 1 073 092

## Description

The present invention concerns the field of the alternative energies and more specifically it refers to a gas container at constant pressure specifically adapted for collecting and stocking any gas generated from waste waters or from biological sludges.

The anaerobic digestion is a microbiologic process by which many organic substances are transformed into methane and carbon dioxide ( the so called biogas). The process occurs by nature in conditions of lock of oxygen and artificially in closed reactors.

The animal dejecta are a very good substrate for the production of bio-gas; to the purpose of producing it in an inexpensive way the cost of the plant should be the lowest possible.

While designing very inexpensive plants during the past years several systems have been developed to cover with plastic material tanks and areas (lagoons) for stocking animal waste waters so that biogas is collected which is formed by nature in aerobic conditions.

Presently, the systems for recovering biogas from animal waste waters stocking lagoons are very rough and can be classified in two main types:
1. Wide arch canvas coverings (balloons) anchored by special systems to the lagoon which are inflated by the gas which is produced. In these systems the gas has a very low pressure and variable as a function of the balloon inflation. The latter because it is not under pressure for most of the time, it is subjected to flap due to wind and tends to form raining water puddles and to be submersed under the snow, to serious detriment ensuring to the plant both with regards to its efficiency and durability.
2. Low canvas coverings with floats. In these systems which are formed by plastic sheets which rest on a liquid surface and are kept lifted at a distance of 1-2 m from a floating cylindrical element, the gas is withdrawn more or less continuously by means of a suction pump. This system is less subjected to wind but anyway it can be submerged by rain or snow and has no stocking capacity.

A purpose of this invention is to overcome the deficiencies of the up to now known systems by means of a plant conforming to the requirements of low cost and good efficiency as needed for the productions and economic exploitment of biogas.

From French Patent N. 1.547.683 it is known a gasometer formed by an outer closed membrane of generally cylindrical form, the inside of which is divided into two chambers by a second membrane separating the volume in two zones containing different gases, i.e. the stocking gas and an auxiliary gas which permits to keep the pressure of the stocking gas constant.

According to this invention, a biogas recovery container is provided which comprises an impermeable membrane which will be called inner membrane interposed between the surface of the waste waters from which the biogas is released and the outer covering membrane, which conventionally is used to collecte said gas. Thereby two chambers are obtained that is the gas build-up chamber underneath the inner membrane and the air chamber included between the inner membrane and the outer membrane, said air chamber being occupied by an auxiliary inflating gas supplied from outside to maintain the pressur constant.

This invention will be now described with reference to the attached drawings by which a preferred embodiment of the invention is shown as an illustration and not as a limitation thereof.

In the drawings:
- Figs. 1 and 2 schematically show the technical teaching on which this invention is based.
- Fig.3 shows the draft according to this invention of a plant for collecting and stocking biogas.
- Fig.3A shows a cross section of the plant of Fig.3.

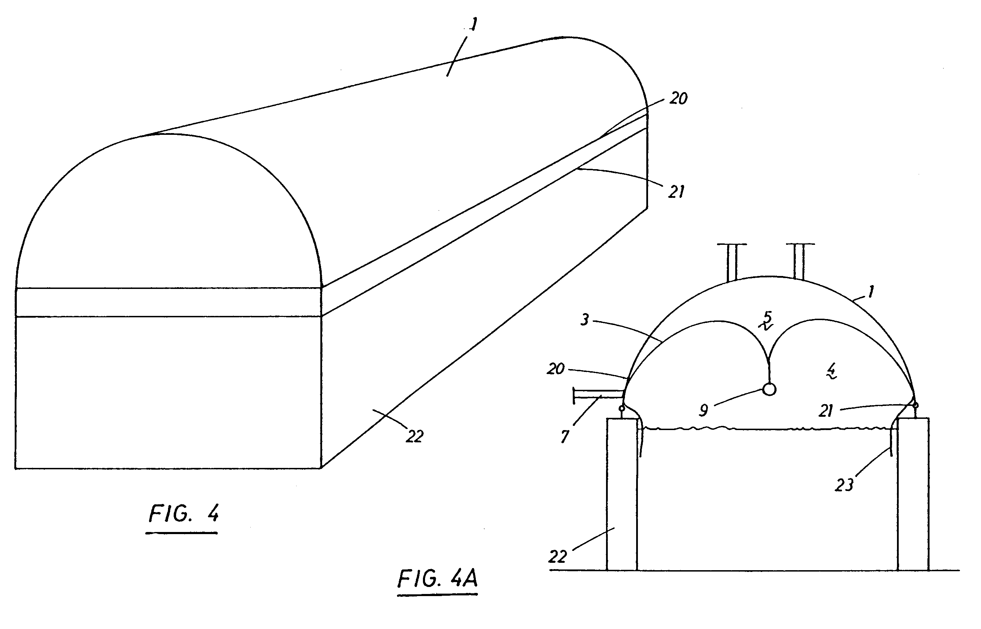

- Fig.4 shows a plant which is anchorated to the bank of a cistern.
- Fig.4A shows a cross section of Fig.4.
- Fig.5 shows the scheme of a governing and pumping switchboard adapted for being installed in said plants.

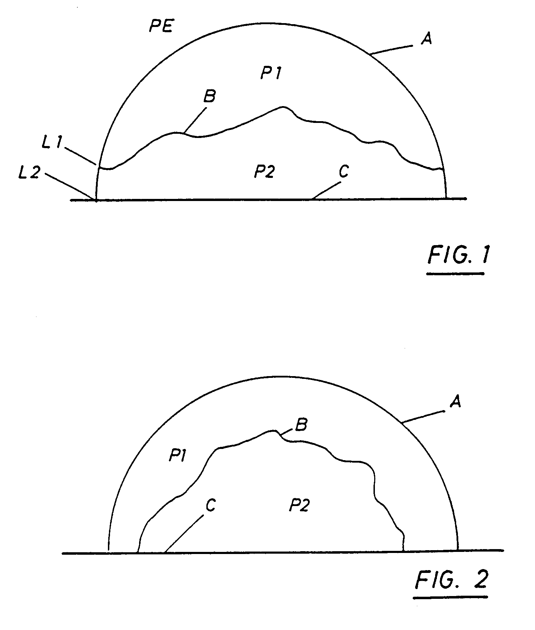

With reference to figures 1-2 this invention substantially includes a container which comprises two flexible membranes A and B which are united together along a closed line L1 so that a chamber P1 is formed the walls of which are said two membranes; at its turn said container is united to a solid or liquid surface C along a closed line L2 so as to form a second chamber P2.

The so obtained two chambers are separated from one another by a common impermeable membrane B.

In some ways, also a portion of surface C can be used as a separating element between chambers P1 and P2 (see Fig.2).

When observing Fig.1, it becomes obvious that when the pressure in chamber P1 is maintained constant by feeding or taking away air and other gases, also the pressure in P2 remains constant with respect to PE which is the atmospheric pressure. A certain amount of gas can therefore be stocked in chamber P2 with no change of pressure within its inside. The system thus becomes a gas container at constant pressure whenever the amount of the contained gas changes and, in addition, it can also display the following functions:
1. If C is a liquid surface from which a gas is released ( animal waste waters, town and industry sewage) chamber P2 can collect them.
2. For suitable pressures within chamber P1, surface A operates as a covering which is effective for standing wind and snow loads.
3. Chamber P1 can be used as a thermal insulator for chamber P2 with respect to the outside and alternately can work as a sun trap for heating the liquid which is covered by the container.
4. The gas which is contained within P2 can be compressed even if it is inflammable and corrosive by means of usual air pumps, in fact it would suffice only to pump air into chamber P1.

From the above it evidently ensues that by this invention the following advantages can be achieved with respect to the preceeding state of the art.
- From liquid surfaces and solid stacks the gases can be collected without mixing them to the air and without any special suction pumps for gases as used up to now.
- The collected gases can be accumulated also in large quantities while maintaining unchanged the outer shape and the endurance to the weather agents whereby the plant can be continuatively used also during the winter (the existing plants are troubled by rain, wind and snow).
- Gases can be delivered at constant pressure at any flow rate.

- The flexible membranes can last longer in as much as:
   a) the outer membrane remains unmovable and uniformly stretched without any damage by variable flexions and strains due to wind and pressure changes;
   b) the inner membrane is totally protected from the weather.
- The production and installation cost is lower when the plant is to be installed on liquid surfaces because no floats are required nor systems for evacuating the rain because chamber P1 is a large float and a totally convex surface.

From this it ensues that this invention can be realized at very low costs.

An operating prototype has been installed and experimented in a lagoon of hoggish waste waters.

The technical characteristics are as follows :
- 20 meters long
- 6 meters wide
- round arch
- material of the outer sheet: Trevira cloth, PVC coated;
- membrane material: coupled PVC;
- operating pressure 70 mm H20 inside the air-chamber and - 75 mm H20 inside the biogas chamber ( the sheet and the counterweight being accounted) for.
- A fan for maintaining the pressure within the chamber of air, which fan is controlled by a pressure automatic regulator and has an absorbed power of 200 W and a maximum flow rate of about 200 m3 per hour.
- An anchoring ballast comprising a peripheral tube of 80 cm diam. made of PVC coated Trevira full of water.
- A high- water mark of the gas chamber which is checked by means of three electromechanic feelers.

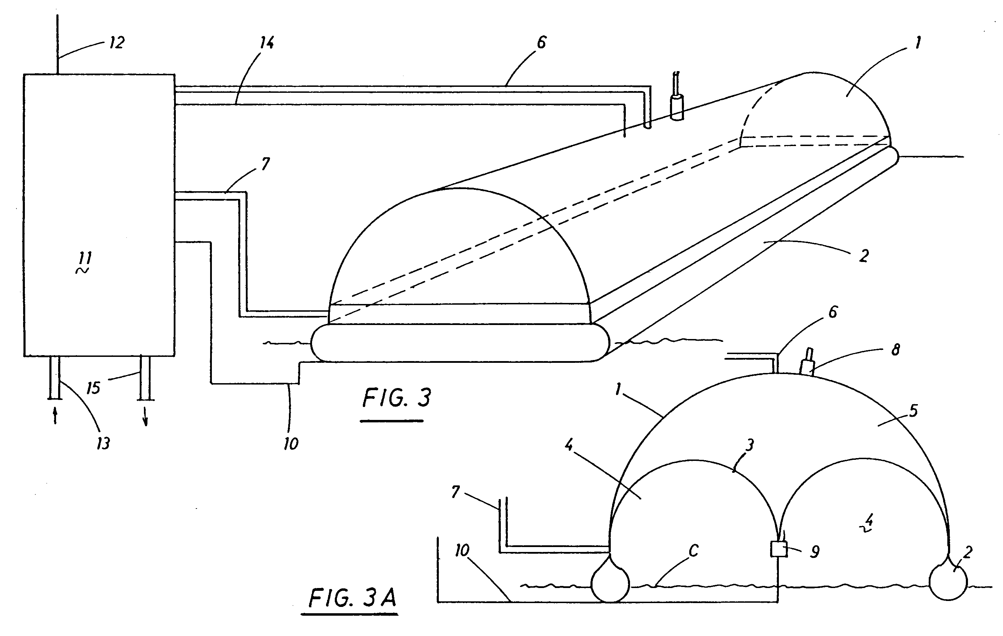

In figures 3 and 3A a plant is illustrated for collecting and stocking the biogas which floats on a liquid surface. Number 1 indicates the outer membrane which comprises also the covering and is connected with the water tube 2 which operates as a counterweight and a seal for the gases which are formed on top of surface C.

Inside covering 1 the inner membrane is located, which divides the volume of the space in two chambers which are respectively a chamber 4 for collecting the biogas and an air-chamber 5 into which air can be fed and from which it can be withdrawn through pipe 6. The biogas is removed by means of duct 7 and at the top of air chamber 5 an overpressure valve 8 is provided which is intended for discharging the air from said chamber whenever the inner pressure overcomes a predetermined value.

As shown in figure 3A, the inner membrane forms two lobes which are determined by the counterweight 9, for the feeler which checks the level of said membrane. Said feeler is connected through wire 10 to switchboard 11 which will be described in detail.

To the switchboard the following are connected:
the electric feeding wire 12, the presurizing air duct 13, the wire 14 of the pressure automatic regulator feeler of the air chamber and the already mentioned duct 7.

From pressure automatic regulator 11, duct 15 sets out for sending biogas to the use.

When the biogas is to be obtained by the material which is located in a cistern-tank, the plant schematically illustrated by drawings 4 and 4A can be used. In this case the outer membrane 1 and the inner membrane 3 are united to one another along the lateral lines 20 and anchored to the sides 22 of the collecting tank. A perimetrical apron 23 is provided for ensuring the seal.

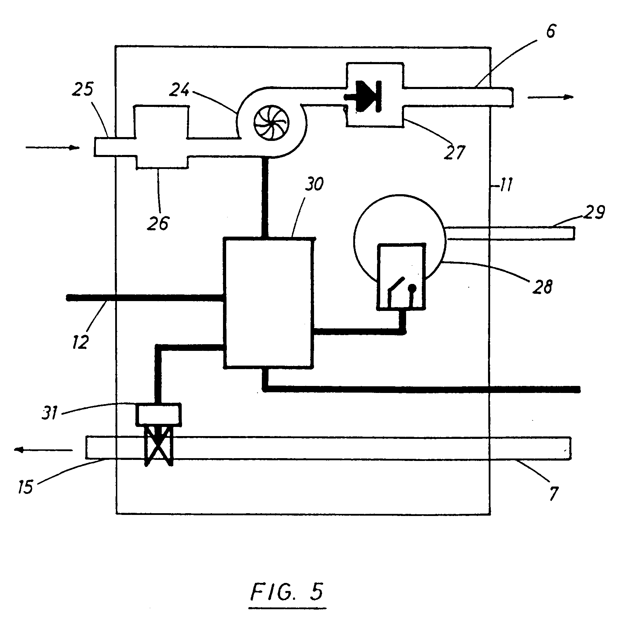

All the above plants are equipped with a regulating and pumping switchboard which is illustrated in detail by figure 5. It includes a centrifugal pump 24 which deducts air from the outside through intake 25 which is associated to filter 26 and sends it to air chamber 5 through duct 6 which is provided with a check valve 27 which cooperates with pressure automatic regulator 28 which is connected to the air-chamber 5 by means of duct 29.

The electric board 30 which is reached by the feeding wire 12, controls pump 24, pressure automatic regulator 28 and electrovalve 31 which is inserted between duct 7 which comes from collecting chamber of biogas and duct 15 by which it is sent to the users. Said board is further provided with a device adapted for showing the amount of biogas contained in the accumulation chamber 4. In figure 5, for better understanding, the electric connections are indicated by a continuous line while the air and gas ducts are indicated by a double line.

The present invention has been illustrated and described in some of its preferred embodiments; however it should be understood that any structural variants could be contributed thereto without departing from the protective scope of the present industrial patent.

## Claims

1. A gas container for storing biogas produced from animal sewage contained in a tank, comprising a tank, a convex outer flexible membrane (A) defining a space above the upper side of the tank, an inner flexible membrane (B) of impermeable type located between an upper surface (C) of the contents of the tank and the outer membrane (A) so to form a lower chamber (P2) defined by said upper surface (C) and said inner membrane (B) occupied by biogas released from the tank, and an upper chamber (P1) defined by said outer membrane (A) and said inner membrane (B) occupied by an auxiliary inflating gas supplied from outside to maintain the pressure constant.

2. A gas container according to claim 1, characterized in that the lower chamber (P2) is delimited by the inner membrane (B,3) and the free surface (C) of the sewage or mud by which the biogas is generated.

3. A gas container according to claim 1 characterized in that the two flexible membranes (A,1;B,3) are joined to one another along a closed line (L1;20).

4. A gas container according to claim 1 characterized in that a portion of the surface (C) of material by which the biogas is generated is used as separating element between the two chambers (P1, P2).

5. A gas container according to claim 1 characterized in that the outer membrane (1) has the base edge anchored to a counter weight (23) made of water or any other inert material immerged into the surface of the biogas source.

6. A gas container according to claim 1 characterized in that the inner membrane (3) forms two lobes which are determined by counterweight (9) for the feeler which checks the level of said membrane.

7. A gas container according to claim 1 characterized in that the outer membrane (1) and the inner membrane (2) are joined to each other along the lateral line (20) of a cistern-tank, where the material is located from which the biogas is released, and anchored to the sides (22) of the collecting tank, a perimetrical apron (23) being provided for ensuring the seal.

## Patentansprüche

1. Gasbehälter zum Speichern von Biogas, das aus in einem Tank enthaltenem tierischen Abwasser erzeugt wird, mit einem Tank, einer konvexen flexiblen Außenmembran (A), die einen Raum über der oberen Seite des Tanks bestimmt, einer flexiblen Innenmembran (B) undurchlässigen Typs, die zwischen der oberen Oberfläche (C) des Tankinhalts und dar Außenmembran (A) derart angeordnet ist, daß eine untere Kammer (P2) bestimmt wird, die durch die obere Oberfläche (C) und die Innenmembran (B) gebildet wird und durch aus dem Tank abgegebenes Biogas eingenommen wird, und einer oberen Kammer (P1), die durch die Außenmembran (A) und die Innenmembran (B) gebildet ist und durch ein zusätzliches Aufblasgas befüllt ist, das von außen zugeführt wird, um den Druck konstant zu halten.

2. Gasbehälter nach Anspruch 1, dadurch gekennzeichnet, daß die untere Kammer (P2) durch die Innenmembran (B,3) und die freie Oberfläche (C) des Abwassers oder des Schlamms begrenzt ist, durch das oder den das Biogas erzeugt wird.

3. Gasbehälter nach Anspruch 1, dadurch gekennzeichnet, daß die zwei flexiblen Membranen (A,1; B,3) miteinander längs einer geschlossenen Linie (L1;20) vereinigt sind.

4. Gasbehälter nach Anspruch 1, dadurch gekennzeichnet, daß ein Abschnitt der Oberfläche (C) des Materials, durch das das Biogas erzeugt wird, als Trennelement zwischen den zwei Kammern (P1,P2) verwendet ist.

5. Gasbehälter nach Anspruch 1, dadurch gekennzeichnet, daß die Außenmembran (1) mit der Basiskante an einem Gegengewicht (23) verankert ist, das aus Wasser oder einem anderen inerten Material besteht, das in die Oberfläche der Biogasquelle eingetaucht ist.

6. Gasbehälter nach Anspruch 1, dadurch gekennzeichnet, daß die Innenmembran (3) zwei Rundbögen bildet, die durch zwei Gegengewichte (9) für den Zähler bestimmt sind, der das Niveau der Membran prüft.

7. Gasbehälter nach Anspruch 1, dadurch gekennzeichnet, daß die Außenmembran (1) und die Innenmembran (2) miteinander längs der Seitenlinie (20) einen Zisternentanks vereinigt sind, wo das Material angeordnet ist, aus dem das Biogas abgegeben wird, und an die Seiten (22) des Sammeltanks verankert sind, wobei eine Begrenzungslinienschürze (23) vorgesehen ist, um die Dichtung sicherzustellen.

## Revendications

1. Un récipient pour gaz permettant de stocker un gaz biologique produit à partir d'effluents animaux contenus dans un réservoir comprenant un réservoir une membrane souple externe convexe (A) définissant un espace au-dessus de la face supérieure du réservoir. une membrane souple interne (B) de type imperméable placée entre une surface supérieure, (C) du contenu du réservoir et la membrane externe (A) afin de former une chambre basse (P2) définie par ladite surface supérieure (C) et ladite membrane interne (B) occupée par le gaz biologique se dégageant du réservoir, et une chambre haute (P1), définie par ladite membrane externe (A) et ladite membrane interne (B), occupée par un gaz de gonflage auxiliaire alimenté de l'extérieur pour maintenir constante la pression.

2. Un récipient pour gaz selon la revendication 1, caractérisé en ce que la chambre basse (P2) est délimitée par la membrane interne (B,3) et la surface libre (C) des effluents ou des boues par lesquels est produit le gaz biologique.

3. Un récipient pour gaz selon la revendication 1, caractérisé en ce que les deux membranes souples (A,1 ; B,3) sont réunies l'une a l'autre le long d'une ligne fermée (L1 ; 20).

4. Un récipient pour gaz selon la revendication 1, caractérisé en ce qu'une partie de la surface (C) de la matière par laquelle est produit le gaz biologique est utilisée comme élément de séparation entre les deux chambres (P1, P2).

5. Un récipient pour gaz selon la revendication 1, caractérisé en ce que la membrane externe (1) présente son bord de base ancré à un contrepoids (23) réalisé en eau ou en une quelconque autre matière inerte immergée dans la surface de la source de gaz biologique.

6. Un récipient pour gaz selon la revendication 1, caractérisé en ce que la membrane interne (3) forme deux lobes qui sont déterminés par un contrepoids (9) pour le palpeur qui contrôle le niveau de ladite membrane.

7. Un récipient pour gaz selon le revendication 1, caractérisé en ce que la membrane externe (1) et la membrane interne (2) sont réunies l'une à l'autre le long de la ligne latérale (20) d'un réservoir formant citerne, où est placée la matière à partir de laquelle le gaz biologique se dégage, et ancrés aux côtés (22) du réservoir de retenue, un tablier périphérique (23) étant prévu pour assurer l'étanchéité.
